(19) Europäisches Patentamt
European Patent Office
Office européen des brevets

(11) Veröffentlichungsnummer: **0 141 387 B1**

# (12) EUROPÄISCHE PATENTSCHRIFT

(45) Veröffentlichungstag der Patentschrift: **12.04.89**

(51) Int. Cl.[4]: **C 07 D 457/12**, C 07 D 457/06, C 07 D 519/02

(21) Anmeldenummer: **84112913.3**

(22) Anmeldetag: **26.10.84**

(54) **Verfahren zur Herstellung von 2-Brom-8-ergolinyl-Verbindungen.**

(30) Priorität: **03.11.83 DE 3340025**

(43) Veröffentlichungstag der Anmeldung: **15.05.85 Patentblatt 85/20**

(45) Bekanntmachung des Hinweises auf die Patenterteilung: **12.04.89 Patentblatt 89/15**

(84) Benannte Vertragsstaaten: **AT BE CH DE FR GB IT LI LU NL SE**

(56) Entgegenhaltungen:
**EP-A- 0 003 667**
**DE-A- 1 926 045**
**FR-A- 2 189 046**
**GB-A- 2 030 567**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Börner, Helmut, Zugdamer Steig 23, D-1000 Berlin 27 (DE)**
Erfinder: **Haffer, Gregor, Dr., Mörchinger Strasse 79, D-1000 Berlin 37 (DE)**
Erfinder: **Sauer, Gerhard, Dr., Königsbacher Zeile 41A, D-1000 Berlin 28 (DE)**

**EP 0 141 387 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 2-Brom-8-ergolinyl-Verbindungen der Formel I

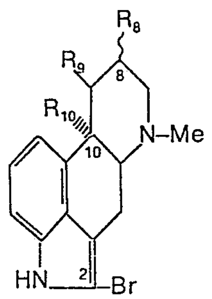

(I),

worin

$R^8$ bedeutet $NH_2$, NH–CO $NEt_2$, $CONH_2$,

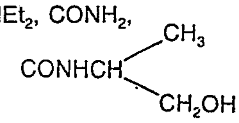

und

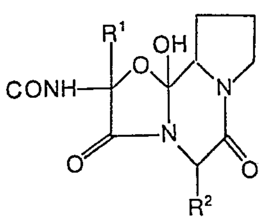

mit

$R^1$ = $C_{1-4}$-Alkyl und

$R^2$ = $C_{1-4}$-Alkyl und Benzyl,

$R^9$, $R^{10}$ jeweils Wasserstoff oder gemeinsam eine Bindung darstellen und der Substituent $R^8$ α- oder β-ständig sein kann und deren Säureadditionssalzen, aus entsprechenden in 2-Stellung unbromierten 8-Ergolinylverbindungen und deren Säureadditionssalzen durch Bromierung, dadurch gekennzeichnet, daß man die Bromierung mit elementarem Brom in Gegenwart von Bromwasserstoff in einem halogenierten Kohlenwasserstoff als Lösungsmittel durchführt und gewünschtenfalls anschließend das Säureadditionssalz herstellt.

2-Brom-8-ergolinyl-Verbindungen sind wichtige Arzneimittel aus der Reihe der Mutterkorn-Alkaloide wie z.B. das bekannte Bromocriptin zur Behandlung von Hyperprolaktinämie oder das 2-Brom-Lisurid (EP-A-0 056 358). Sie sind aber auch als Zwischenprodukte zur Herstellung von pharmakologisch wirksamen Ergot-Alkaloiden verwendbar.

Bromierungsverfahren für Ergotalkaloide sind seit längerem bekannt (vgl. z.B. F. Troxler und A. Hofmann, Helv. Chim. Acta 40 (1957) 2160). Nach diesem klassischen Verfahren werden Lysergsäurederivate in der Wärme mit 1,2 bis 1,5 Moläquivalenten N-Bromsuccinimid in Dioxan zu den entsprechenden 2-Bromverbindungen mit mittlerer Ausbeute erhalten. Es hat in der Vergangenheit nicht daran gefehlt, die verschiedensten Bromierungsmittel wie Dioxandibromid, N-Bromcaprolactam oder N-Bromphthalimid auszuprobieren (z.B. DE-A-1 926 045).

Andere selektive Bromierungsmittel für diesen Zweck sind z.B. die Bromadditionskomplexe Pyrrolidon-(2)-hydrotribromid (DE-A-2 752 532) und 3-Brom-6-chlor-2-methylimidoazo[1.2-b]pyridazin-hydrotribromid (DE-A-2 938 313).

Ferner ist in der FR-A-2 189 046 ein Verfahren zur Bromierung von Ergolinen in 2,13-Stellung beschrieben, wonach Eisessig als Reaktionsmedium unter Zugabe von elementarem Brom und Bromwasserstoff verwendet wird.

Alle diese Bromierungsverfahren haben den Nachteil, daß einmal die Ausbeuten an gewünschtem Bromierungsprodukt nicht quantitativ sind und zum anderen das Bromierungsprodukt oft technisch recht aufwendig von den entsprechenden aus den verwendeten Bromierungsreagenzien entstandenen, im Reaktionsgemisch sich befindenden Trägerverbindungen abgetrennt werden müssen.

Die vorliegende Erfindung hatte die Aufgabe, ein selektives Bromierungsverfahren für 8-Ergolinylverbindungen bereitzustellen, das nahezu quantitative Ausbeuten liefert und das die Aufarbeitung des Bromierungsprodukts technisch vereinfacht.

Die erfindungsgemäße Aufgabe wurde dadurch gelöst, daß die Bromierungsreaktion mit elementarem Brom in Gegenwart von Bromwasserstoff in einem halogenierten Kohlenwasserstoff als Lösungsmittel ausgeführt wird.

Als halogenierte Kohlenwasserstoffe seien beispielsweise Methylenchlorid, Tetrachlorkohlenstoff, Chloroform, 1.2-Dichlorethan, 1.2-Trichlortrifluorethan und insbesondere Methylendichlorid genannt.

Die erfindungsgemäße Reaktion wird zweckmäßigerweise unter Schutzgas wie Stickstoff- oder Edelgasatmosphäre und bei Temperaturen unter Raumtemperatur, vorzugsweise bei 0–15 °C, d.h. bei Temperaturen, wie sie durch äußere Eiswasserkühlung erreicht werden, ausgeführt.

Das Ausgangsmaterial kann in freier Form, d.h. als freie Verbindung oder auch in Form eines Salzes, verwendet werden.

Als Salze seien beispielsweise genannt das Hydrogenmaleat, Hydrogenphosphat, Methansulfonat, Hydrochlorid, Hydrobromid, Hydrogensulfat und Tartrat.

Das elementare Brom sowie der Bromwasserstoff werden in etwa äquimolaren Mengen eingesetzt, wobei ein geringer Überschuß angewendet wird. Es werden somit 1,0 bis 1,1 Moläquivalente Brom bzw. Bromwasserstoff bezogen auf die 8-Ergolinylverbindung verwendet.

Der Bromwasserstoff wird hierzu zweckmäßigerweise in Eisessig verdünnt, wobei sich eine 33%ige Lösung von Bromwasserstoff in Eisessig gut bewährt hat.

Das Reaktionsprodukt kann anschließend durch einfache technische Maßnahme wie Extraktion und Kristallisation vom Reaktionsgemisch abgetrennt werden.

Der Verlauf des erfindungsgemäßen Verfahrens war insofern überraschend, als bei den Arbeiten von Troxler und Hofman (l.ct.) auch bereits molekulares Brom verwendet worden war und man sehr rasch ein Gemisch verschiedener überbromierter Verbindungen erhalten hatte, welches darüber hinaus noch beträchtliche Mengen von Zersetzungsprodukten enthielt, von denen sich

nur sehr schwer einheitliche Derivate abtrennen ließen.

Es war daher nicht vorhersehbar, daß man beim Bromieren mit molekularem Brom in Gegenwart von einer gleichen Menge Bromwasserstoff in einem halogenierten Kohlenwasserstoff als Lösungsmittel selektiv in 2-Stellung der 8-Egolinylverbindungen halogeniert, wobei man zu einem Reaktionsgemisch gelangt, das sich relativ einfach aufarbeiten läßt.

Die Bromierung verläuft regioselektiv in 2-Stellung. Eine Epimerisierung in 8-Stellung tritt nicht auf.

Hieraus resultiert, daß entsprechend hohe Ausbeuten an den gewünschten 2-Brom-8-ergolinylverbindungen erhalten werden.

Die nachfolgenden Beispiele sollen das erfindungsgemäße Verfahren erläutern.

Beispiel 1
(Herstellung von 2-Brom-Lisurid):

22,73 g 3-(9.10-Didehydro-6-methyl-8α-ergolinyl)-1.1-diethylharnstoff, Hydrogenmaleinat (= Lisuridhydrogenmaleat) 50 mmol werden in 500 ml Methylendichlorid unter Stickstoffatmosphäre suspendiert. Unter Kühlung des Ansatzes mit Eiswasser und Rühren tropft man 9,48 ml Bromwasserstoff in Eisessig (33%ig, 55 mMol) innerhalb 1 Minute zu. Die Substanz löst sich während der Zugabe auf. Die Reaktionslösung färbt sich grün. Anschließend gibt man während 2 Stunden und weiterer Kühlung 2,68 ml Brom (52,5 mMol), in 500 ml Methylendichlorid gelöst, gleichäßig hinzu, rührt 10 Minuten nach, verdünnt mit 1000 ml Methylendichlorid, versetzt anschließend mit 750 ml 5%iger Natriumhydrogencarbonatlösung und läßt innerhalb von 30 Minuten unter Rühren auf Raumtemperatur erwärmen. Die Methylendichloridphase wird abgetrennt, die wäßrige Phase dreimal mit je 500 ml Methylendichlorid extrahiert. Die vereinigten Methylendichloridlösungen werden einmal mit 500 ml Wasser gewaschen und das Waschwasser mit 250 ml Methylendichlorid nachextrahiert. Die über Natriumsulfat getrocknete Methylendichloridlösung wird im Vakuum eingedampft und 4 Stunden bei −15 °C zur Kristallisation gebracht. Der Niederschlag wird abgesaugt, mit 20 ml Methylendichlorid gewaschen und im Vakuum getrocknet. Man erhält 22,90 g Rohprodukt, von welchem 22,80 g in 2000 ml Methylenchlorid gelöst und 30 Minuten mit 34 g Kieselgel gerührt werden. Der über eine Fritte gegebene Feststoff wird nacheinander mit 1000 ml Methylendichlorid und 1000 ml Methylendichlorid-Methanol (97:3) gewaschen und getrennt eingedampft. Der Rückstand wird in 179 ml Ethanol aufgenommen und unter Rühren mit 119 ml Wasser versetzt. Der Niederschlag wird abgesaugt und mit 15 ml kaltem Ethanol-Wasser-Gemisch (60:40) gewaschen und getrocknet. Man erhält 11,51 g 2-Brom-Lisurid.

Die Mutterlauge wird im Vakuum eingeengt, dann dreimal mit je 50 ml Methylendichlorid ausgeschüttelt und zur Trockne eingedampft. Der Rückstand wird zusammen mit dem Methylendichlorid-Methanol-Rückstand an 250 g Kieselgel mit 2400 ml Methylendichlorid-Methanol (97:3) eluiert, eingedampft, in 82,5 ml Ethanol gelöst und nach Zugabe von 55 ml Wasser wie oben angegeben aufgearbeitet. Man erhält weitere 6,16 g 2-Brom-Lisurid. Gesamtausbeute: 17,67 g (85,0% d.Th.); Schmelzpunkt: 133–140 °C (Zers.), $[\alpha]_D^{25}$ = +305,2° (c = 0,5 Pyridin).

In gleicher Weise erhält man aus dem freien
3-(9.10-Didehydro-6-methyl-8α-ergolinyl)-1.1-diethylharnstoff und aus dem
3-(9.10-Didehydro-6-methyl-8α-ergolinyl)-1.1-diethylharnstoff-Hydrobromid (Schmelzpunkt 221 °C (Zers.); $[\alpha]_D^{25}$ = +227,2° (c = 0,5, Pyridin)) das
2-Brom-3-(9.10-Didehydro-6-methyl-8α-ergolinyl)-1.1-diethylharnstoff, (d.h. 2-Brom-Lisurid) in einer Ausbeute von 8,43% d.Th. ($[\alpha]_D^{25}$ = +305,0° (c = 0,5, Pyridin)) bzw. 81,5% d.Th. ($[\alpha]_D^{25}$ = 304,7° (c = 0,5, Pyridin)).

Beispiel 2
(Herstellung des Hydrobromids von 2-Brom-Lisurid):

417,4 mg 2-Brom-Lisurid (1 mMol) werden in 8,5 ml Ethanol gelöst. Unter Inertgasatmosphäre tropft man bei Raumtemperatur innerhalb 1 Minute 0,189 ml Bromwasserstoff in Eisessig (33%ig, 1,1 mMol) zu, rührt 10 Minuten nach und läßt eiswassergekühlt kristallisieren. Der abfiltrierte Niederschlag wird mit wenig eiskaltem Ethanol nachgewaschen. Man erhält 433,5 mg 2-Brom-Lisurid-Hydrobromid (87,0% d.Th.), Schmelzpunkt 225–230 °C (Zers.), $[\alpha]_D^{25}$ = +311,8° (c = 0,5, Pyridin).

Beispiel 3
(Herstellung verschiedener 2-Brom-8-ergolinyl-Verbindungen der Formel I):

Analog Beispiel 1 erhält man aus dem
3-(9.10-Didehydro-6-methyl-8β-ergolinyl)-1.1-diethyl-harnstoff
den
3-(2-Brom-9.10-didehydro-6-methyl-8β-ergolinyl)-1.1-diethyl-harnstoff;
74,8% Ausbeute, $[\alpha]_D^{25}$ = +104,1° (c = 0,5, Pyridin) = +45,3° (c = 0,5, Methanol)
Hydrobromid; 84,0% Ausbeute, $[\alpha]_D^{25}$ = 39,2° (c = 0,5, Methanol) = +55,0° (c = 0,5, Pyridin);
aus
1.1-Diethyl-3-(6-methyl-8α-ergolinyl)-harnstoff
den
3-(2-Brom-6-methyl-8α-ergolinyl)-1.1-diethylharnstoff;
83,1% Ausbeute, Schmelzpunkt 189–200 °C (Zers.), $[\alpha]_D^{25}$ = 33,3° (c = 0,5, Pyridin)
Hydrobromid; 83,0% Ausbeute, $[\alpha]_D^{25}$ = +60,6° (c = 0,5, Pyridin);
aus
1.1-Diethyl-3-(6-methyl-8β-ergolinyl)-harnstoff
den
3-(2-Brom-6-methyl-8β-ergolinyl)-1.1-diethylharnstoff;
81,4% Ausbeute, $[\alpha]_D^{25}$ = −70,0° (c = 0,5, Pyridin)

Hydrobromid; 82,8% Ausbeute, $[\alpha]_D^{25} = -42,0°$ (c = 0,5, Pyridin);
aus
9.10-Didehydro-6-methyl-ergolin-8α-amin das
2-Brom-9.10-didehydro-6-methyl-ergolin-8α-amin;
46,3% Ausbeute, Schmelzpunkt 245 °C (Zers.), UV (Methanol): $\lambda_{max}$ (ε) = 227 (21 900), 241 (22 900), 303 nm (9460 l/mol · cm);
aus
6-Methyl-ergolin-8α-amin das
2-Brom-6-methyl-ergolin-8α-amin; 68,7% Ausbeute, Schmelzpunkt 242° (Zers.), $[\alpha]_D^{25} = -63,5°$ (c = 0,5, Pyridin);
aus
9.10-Didehydro-6-methyl-ergolin-8α-carbonsäureamid
das
2-Brom-9.10-didehydro-6-methyl-ergolin-8α-carbonsäureamid,
55,0% Ausbeute, Schmelzpunkt 213 °C (Zers.), $[\alpha]_D^{25} = +447°$ (c = 0,5, Pyridin);
aus
6-Methyl-ergolin-8α-carbonsäureamid das
2-Brom-6-methyl-ergolin-8α-carbonsäureamid, 78,0% Ausbeute, Schmelzpunkt 248–252 °C (Zers.), $[\alpha]_D^{25} = -1,0°$ (c = 0,5, Pyridin);
aus
9.10-Didehydro-6-methyl-8β-ergolincarbonsäure-(1S)-(1-hydroxy-methylethyl)-amid-hydrogenmaleinat (Ergometrinhydrogenmaleinat)
das
2-Brom-ergometrin; 74,5% Ausbeute, Schmelzpunkt 142 °C (Zers.), $[\alpha]_D^{25} = -13,8°$ (c = 0,5, Pyridin);
6-Methyl-8β-ergolincarbonsäure-(1S)-1-hydroxymethylethyl)-amid (Dihydroergometrin)
das
2-Brom-dihydroergometrin; 81,4% Ausbeute, Schmelzpunkt 220–225 °C (Zers.), $[\alpha]_D^{25} = -131,8°$ (c = 0,5, Pyridin);
aus
(5′α)-12′-hydroxy-2′-methyl-5′-benzyl-ergotamin-3′.6′.18-trion-tartrat (Ergotamin-tartrat)
das
2-Brom-ergotamin; 68,0% Ausbeute, Schmelzpunkt 195 °C (Zersetzung), $[\alpha]_D^{25} = -160,7°$ (c = 0,5, Chloroform) = −19,4° (c = 0,5, Pyridin);
aus
9.10-Dihydroergotamin das
2-Brom-9.10-dihydroergotamin; 76,2% Ausbeute, Schmelzpunkt 199–201 °C (Zers.), $[\alpha]_D^{25} = -87,8°$ (c = 0,5, Pyridin),
und aus
(5′α)-12′-hydroxy-2′-(1-methylethyl)-5′-(2-methylpropyl)-ergotamin-3′.6′.18-trion (α-Ergocryptin)
das
2-Brom-α-ergocryptin; 73,5% Ausbeute, Schmelzpunkt 213 °C )Zers.), $[\alpha]_D^{25} = -95,5°$ (c = 0,5, Pyridin) = −189,3° (c = 0,5, Chloroform).

## Patentanspruch

Verfahren zur Herstellung von 2-Brom-8-ergolinyl-Verbindungen der Formel I

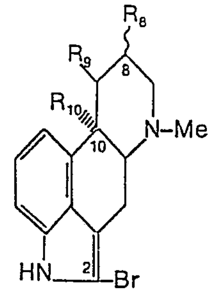

(I),

worin
$R^8$ bedeutet $NH_2$, NH–CO $NEt_2$, $CONH_2$,

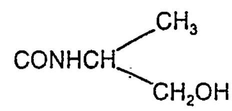

und

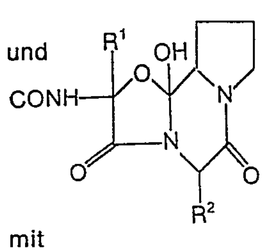

mit
$R^1$ = $C_{1-4}$-Alkyl und
$R^2$ = $C_{1-4}$-Alkyl und Benzyl,
$R^9$, $R^{10}$ jeweils Wasserstoff oder gemeinsam eine Bindung darstellen und der Substituent $R^8$ α- oder β-ständig sein kann und deren Säureadditionssalzen, aus entsprechenden in 2-Stellung unbromierten 8-Ergolinylverbindungen und deren Säureadditionssalzen durch Bromierung, dadurch gekennzeichnet, daß man die Bromierung mit elementarem Brom in Gegenwart von Bromwasserstoff in einem halogenierten Kohlenwasserstoff als Lösungsmittel durchführt und gewünschtenfalls anschließend das Säureadditionssalz herstellt.

## Claim

A process for the preparation of 2-bromo-8-ergolinyl compounds of the formula I

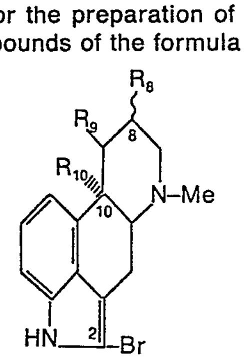

(I),

wherein
$R^8$ is $NH_2$, NH–$CONEt_2$, $CONH_2$,

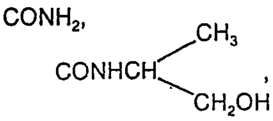

,

and

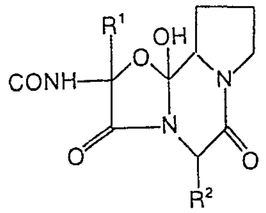

wherein

$R^1$ is $C_{1-4}$-alkyl, and

$R^2$ is $C_{1-4}$-alkyl and benzyl,

$R^9$ and $R^{10}$ each are hydrogen or together form a bond, and the substituent $R^8$ may be in the α- or β-position, and the acid additions salts thereof,

from corresponding 8-ergolinyl compounds that are unbrominated in the 2-position, and acid addition salts thereof, by bromination, characterised in that the bromination is carried out with elemental bromine in the presence of hydrogen bromide, in a halogenated hydrocarbon as solvent, and, if desired, the acid addition salt is subsequently prepared.

**Revendication**

Procédé pour préparer des composés bromo-2-ergolinyliques-8 répondant à la formule I:

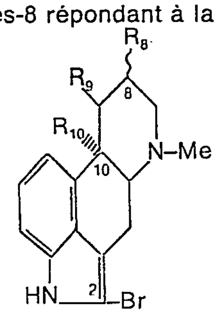

(I)

dans laquelle

$R^8$ représente un radical $-NH_2$, $-NH-CONEt_2$, $-CONH_2$ ou

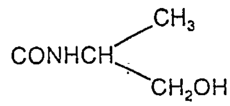

ou un radical de formule:

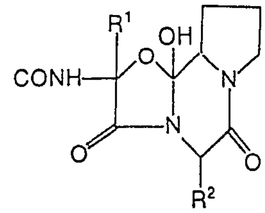

dans lequel

$R^1$ désigne un alkyle en $C_1-C_4$ et

$R^2$ un alkyle en $C_1-C_4$ ou un benzyle,

$R^8$ et $R^{10}$ représentent chacun l'hydrogène ou forment ensemble une liaison,

et le substituant $R^8$ a la configuration α ou la configuration β,

et leurs sels d'addition d'acides, à partir des composés ergolinyliques-8 non bromés en position 2 correspondants et de leurs sels d'addition d'acides, par bromation, procédé caractérisé en ce que la bromation est effectuée par du brome élémentaire, en présence de bromure d'hydrogène, dans un hydrocarbure halogéné servant de solvant, et en ce qu'on prépare ensuite, si on le désire, le sel d'addition d'acide.